(19) Europäisches Patentamt
European Patent Office
Office européen des brevets

(11) Veröffentlichungsnummer: **0 478 941 B1**

(12) # EUROPÄISCHE PATENTSCHRIFT

(45) Veröffentlichungstag der Patentschrift: **01.02.95**

(21) Anmeldenummer: **91114115.8**

(22) Anmeldetag: **23.08.91**

(51) Int. Cl.⁶: **A61K 31/215**, A61K 31/275, A61K 31/04, A61K 31/165, A61K 31/66, A61K 31/195, A61K 31/13, A61K 31/40, A61K 31/35

(54) **Arzneimittel welche substituierte 2-Cyclohexen-1yl-amin-Derivate enthalten und ihre Verwendung zur Bekämpfung von Krankheiten.**

(30) Priorität: **05.09.90 DE 4028046**

(43) Veröffentlichungstag der Anmeldung:
**08.04.92 Patentblatt 92/15**

(45) Bekanntmachung des Hinweises auf die Patenterteilung:
**01.02.95 Patentblatt 95/05**

(84) Benannte Vertragsstaaten:
**AT BE CH DE DK ES FR GB GR IT LI LU NL SE**

(56) Entgegenhaltungen:
**EP-A- 0 258 853** **EP-A- 0 376 072**
**EP-A- 0 445 749** **DE-A- 2 150 516**
**DE-A- 2 243 803** **DE-A- 3 126 818**
**FR-A- 2 014 957** **US-A- 3 882 164**

**JOURNAL OF MEDICINAL CHEMISTRY, Band 24, Nr. 7, Juli 1981, Seiten 788-794, American Chemical Society, Washington, US; D.T. WITIAK et al.: "Epimeric cis-Decahydroquinoline-5-carboxylic acids: Effects on gamma-aminobutyric acid uptake and receptor binding in vitro"**

(73) Patentinhaber: **BAYER AG**

**D-51368 Leverkusen (DE)**

(72) Erfinder: **Kunisch, Franz, Dr.**
**Zum Hahnenberg 20**
**W-5068 Odenthal (DE)**
Erfinder: **Babczinski, Peter, Dr.**
**In der Lohrenbeck 11**
**W-5600 Wuppertal (DE)**
Erfinder: **Arlt, Dieter, Prof. Dr.**
**Rybniker Strasse 2**
**W-5000 Köln 80 (DE)**
Erfinder: **Plempel, Manfred, Dr.**
**Zwengenberger Strasse 3c**
**W-5657 Haan (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung bezieht sich auf die Verwendung von bekannten substituierten 2-Cyclohexen-1-yl-amin-Derivaten bei der Bekämpfung von Krankheiten, insbesondere bakteriellen Infekten und Mykosen.

Die erfindungsgemäßen Verbindungen sind in der europäischen Patentanmeldung EP-0 376 072 A2 ausführlich beschrieben.

Es wurde nun gefunden, daß die substituierten 2-Cyclohexen-1-yl-amin-Derivate der Formel (I)

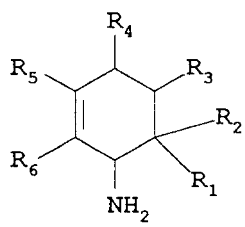

(I),

in welcher

$R^1$ — für Wasserstoff, geradkettiges oder verzweigtes Alkyl mit 1 bis 6 Kohlenstoffatomen oder Fluor, Chlor oder Brom steht,

$R^2$ — für Formyl, geradkettiges oder verzweigtes Hydroxyalkyl mit 1 bis 8 Kohlenstoffatomen im Alkylteil, Cyano, Nitro oder für einen der Reste

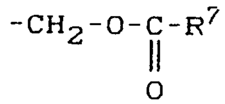 , 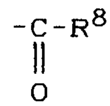

oder $-CH=CH-R^9$ steht,

$R^3$, $R^4$, $R^5$ und $R^6$ — gleich oder verschieden sind und jeweils für Wasserstoff, jeweils geradkettiges oder verzweigtes Alkyl oder Alkoxy mit 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, Alkoxyalkyloxy mit jeweils 1 bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, $C_1$-$C_4$-Alkyl, $C_1$-$C_4$-Alkoxy, $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino,
weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Halogen, Nitro, Cyano, Amino, $C_1$-$C_4$-Alkyl, $C_1$-$C_4$-Alkoxy oder $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino, weiterhin für Fluor, Chlor oder Brom stehen,
wobei mindestens zwei der Reste $R^3$, $R^4$, $R^5$ oder $R^6$ für Wasserstoff stehen,

$R^7$ — für jeweils geradkettiges oder verzweigtes Alkyl oder Alkoxy mit 1 bis 6 Kohlenstoffatomen steht,

$R^8$ — für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, unsubstituiertes oder einfach bis mehrfach, gleich oder verschieden durch Halogen substituiertes Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine Gruppe -O-Z-$NR^{11}R^{12}$, $-NHR^{10}$, $-NR^{11}R^{12}$ oder -OM steht,

$R^9$ für Formyl, Cyano oder für die Gruppe

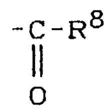

steht,

$R^{10}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{11}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{12}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten infrage kommen,

M für Wasserstoff oder für ein Äquivalent eines entsprechenden Alkalimetall-, Erdalkalimetall- oder Ammoniumkations steht und

Z für eine geradkettige oder verzweigte Alkylkette mit 1 bis 8 Kohlenstoffatomen steht

oder

$R^2$ und $R^3$ gemeinsam für einen der Reste

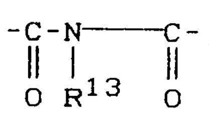,
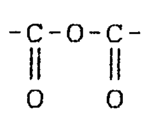
oder
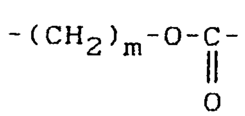,

verbrückt über die Positionen 6 und 5, stehen,

worin

$R^{13}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen und

m für eine Zahl 1 oder 2 steht,

oder

$R^4$ und $R^5$ gemeinsam für eine Alkylkette mit 3- oder 4-Kohlenstoffatomen steht, die über die Positionen 4 und 3 verbunden ist,

sowie deren Säureadditions-Salze und Metallsalz-Komplexe starke antimikrobielle Eigenschaften, im besonderen starke antibakterielle und antimykotische Eigenschaften aufweisen.

Die Verbindungen der Formel (I) können als geometrische Isomere (E/Z-Isomere) oder Isomerengemische unterschiedlicher Zusammensetzung vorliegen. Die Verwendung sowohl der reinen Isomeren als auch der Isomerengemische werden erfindungsgemäß beansprucht. Die Verbindungen der Formel (I) enthalten außerdem 1 bis 4 Chiralitätszentren und können somit in verschiedenen Enantiomeren- und Diastereomerengemischen vorliegen, die gegebenenfalls in üblicher Art und Weise getrennt werden können. Die Verwendung sowohl der reinen Enantiomeren und Diastereomeren, als auch die der Gemische werden ebenfalls erfindungsgemäß beansprucht.

Im folgenden wird der Einfachheit halber stets von der Verwendung von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.

Bevorzugt erfindungsgemäß zu verwendende Verbindungen sind auch Additionsprodukte aus Säuren und denjenigen substituierten 2-Cyclohexen-1-yl-amin-Derivaten der Formel (I) in denen $R^1$, $R^2$, $R^3$ $R^4$, $R^5$ und $R^6$ diejenigen Bedeutungen haben, die im Zusammenhang mit der Beschreibung der erfindungsgemäß zu verwendenden Stoffe bereits für diese Substituerten genannt wurden.

Zu den Säuren, die addiert werden können, gehören vorzugsweise Halogenwasserstoffsäuren, wie z.B. die Chlorwasserotoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Trifluoressigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide, wie z.B. Phthalimid, Saccharin und Thiosaccharin.

Außerdem bevorzugt erfindungsgemäß zu verwendende Verbindungen sind auch Additionsprodukte aus Salzen von Metallen der I., II. und III. Hauptgruppe sowie des Zinns, sowie ferner Salze von Metallen der I., II., VII. und VIII. Nebengruppe des Periodensystems der Elemente und denjenigen substituierten 2-Cyclohexen-1-yl-amin-Derivaten der Formel (I), in denen $R^1$, $R^2$, $R^3$ $R^4$, $R^5$ und $R^6$ die Bedeutungen haben, die bereits im Zusammenhang mit der Beschreibung der erfindungsgemäß zu verwendenden Stoffe der Formel (I) für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Man erhält die substituierten 2-Cyclohexen-1-yl-amin-Derivate der Formel (I), wenn man

A) 2-Cyclohexen-1-yl-carbonsäurederivate der Formel (II)

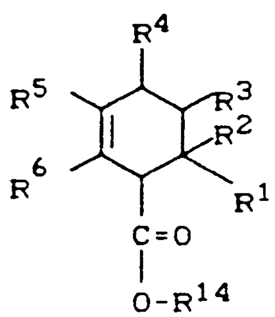

(II)

in welcher

| | |
|---|---|
| $R^1$ , $R^2$ , $R^3$ , $R^4$ , $R^5$ und $R^6$ | die oben angegebene Bedeutung haben und |
| $R^{14}$ | für Wasserstoff, Methyl oder Ethyl steht, |

in allgemein üblicher Weise, nach Curtius, gegebenenfalls in Gegenwart eines Verdünnungsmittels, wie beispielsweise Aceton und in Gegenwart einer Base, wie beispielsweise N,N-Diisopropylamin, bei Temperaturen zwischen -15 °C und +10 °C, mit Chlorameisensäureester versetzt und zu dieser Reaktionsmischung gegebenenfalls in Gegenwart eines Verdünnungsmittels, wie beispielsweise Wasser, bei Temperaturen zwischen -5 °C und +25 °C ein Azid, wie beispielsweise Natriumazid, zugibt, und das intermediär auftretende Isocyanat der Formel (IIa)

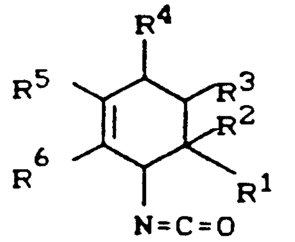

(IIa)

in welcher

$R^1$ , $R^2$ , $R^3$ , $R^4$ , $R^5$ und $R^6$ die oben angegebene Bedeutung haben,

mit Wasser, gegebenenfalls in Gegenwart einer Säure oder einer Base, hydrolysiert und die so erhaltenen Amine gegebenenfalls in Säureadditions-Salze und Metallsalz-Komplexe überführt [vgl. J. Org. Chem. 26, (1961), 3511].

Man erhält die substituierten 2-Cyclohexen-1-yl-amin-Derivate der Formel (I) außerdem, wenn man

B) aus den 2-Cyclohexen-Derivaten der Formel (IIb)

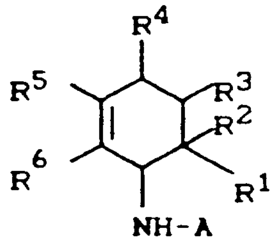

(IIb)

in welcher

$R^1$ , $R^2$ , $R^3$ , $R^4$ , $R^5$ und $R^6$ die oben angegebene Bedeutung haben und

A für eine Aminoschutzgruppe steht,

in an sich bekannter weise nach üblichen Methoden z.B. durch Solvolyse, wie Hydrolyse, Acidolyse, durch Reduktion, wie z.B. durch Hydrogenolyse in Gegenwart eines Hydrierungskatalysators oder mittels eines Reduktionssystems aus Metall und protonenabspaltendem Mittel, wobei je nach Art der Schutzgruppe verschiedenartige (auch andersartige) sowie selektive Abspaltungsmethoden angewendet werden können, gegebenenfalls in Gegenwart eines geeigneten Lösungs- oder Verdünnungsmittels oder eines Gemisches aus ihnen, wobei man je nach Bedarf unter Kühlen, bei Raumtemperatur oder unter Erwärmen, z.B. in einem Temperaturbereich von etwa -10 °C bis zur Siedetemperatur des Reaktionsmediums, vorzugsweise von etwa -10 °C bis etwa 150 °C, und,

falls erforderlich, in einem geschlossenen Gefäss, unter Druck, in einer Inertgasatmosphäre und/oder unter wasserfreien Bedingungen arbeitet und die so erhaltenen Produkte gegebenenfalls in Säureadditions-Salze oder Metallsalz-Komplexe überführt (vgl. Protective Groups in Organic Synthesis, Th. W. Greene, Wiley Interscience, 1981).

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte Reaktion an anderen Stellen des Moleküls durchgeführt worden ist. Typisch für solche Gruppen sind insbesondere unsubstituierte oder substituierte Acyl-, Aryl- z.B. DNP (2,4-Dinitrophenyl), Aralkoxymethyl- z.B. BOM (N-(benzyloxy)methyl) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1 - 20, insbesondere 1 - 8 Kohlenstoffatomen. Der Ausdruck "Acylgruppe" ist im Zusammenhang mit der vorliegenden Erfindung in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA (Phenoxyacetyl); Alkoxycarbonyl wie Methoxycarbonyl, Ethoxycarbonyl, 2,2,2-Trichlorethoxycarbonyl, BOC (tert.-Butoxycarbonyl), 2-Iodethoxycarbonyl; Aralkyloxycarbonyl wie CBZ ("Carbobenoxy") und 4-Methoxybenzyloxycarbonyl. Bevorzugte Aminoschutzgruppen

sind Benzyl, Acetyl, Methoxycarbonyl, Allyloxycarbonyl, Trichlorethyloxycarbonyl, (±)-Menthyloxycarbonyl, tert-Butoxycarbonyl und Benzyloxycarbonyl.

Die vorne u.a. als Aminoschutzgruppe erwähnte Formyl-, Acetyl- oder 2,2,2-Trichloracetyl-gruppe kann beispielsweise durch Hydrolyse abgespalten werden.

Die Hydrolyse erfolgt in an sich bekannter Weise mit Hilfe von Wasser, wobei vorteilhaft in Gegenwart einer die Hydrolyse unterstützenden Säure oder Base, gegebenenfalls in Gegenwart eines inerten Lösungsmittels oder Verdünnungsmittels und/oder unter Kühlen oder Erwärmen gearbeitet wird.

Als Säuren kommen beispielsweise anorganische Säuren, wie Mineralsäuren, z.B. Schwefelsäure, Phosphorsäure oder Halogenwasserstoffsäuren, organische Carbonsäuren, wie Niederalkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder Sulfonsäuren, wie $C_1$-$C_7$-Alkan- oder gegebenenfalls substituierte Benzolsulfonsäure, z.B. Methan- oder p-Toluolsulfonsäure, in Betracht.

Als Basen kommen beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalinamine, $C_1$-$C_7$-Alkylamine, basische Heterocyclen, Ammoniumhydroxide sowie carbocyclische Amine in Frage. Beispielhaft seien Lithium-hydroxid, Natrium-hydroxid, -hydrid, -amid, -ethylat, Kalium-tert-butylat, -carbonat, Lithiumtriphenylmethylid, -diisopropylamid, Kalium-3-(aminopropyl)-amid, -bis-(trimethylsilyl)-amid, Dimethyl-aminonaphthalin, Di- oder Triethylamin, Pyridin, Benzyltrimethyl-ammoniumhydroxid, 1,5-Diazabicyclo-[4.3.0]non-5-en (DBN) sowie 1,8-Diaza-bicyclo-[5.4.0]undec-7-en (DBU) genannt.

Die Acidolyse gelingt z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser.

Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure vorzugsweise in Form eines Gemisches aus Essigsäure und 70 %iger Parchlorsäure im Verhältnis 9 : 1. Die Reaktionstemperaturen für diese Solvolysen liegen zweckmäßig zwischen etwa 0 und etwa 50 °C, vorzugsweise arbeitet man zwischen 15 und 30 °C (Raumtemperatur).

Die BOC-Gruppe kann z.B. bevorzugt mit 40 %iger Trifluoressigsäure in Methylenchlorid oder mit etwa 3 bis 5 n Salzsäure in Dioxan bei 15 - 30 °C abgespalten werden, die FMOC-Gruppe (9-Fluorenylmethyloxycarbonyl) mit einer etwa 5-bis 20%igen Lösung von Dimethylamin, Diethylamin oder Piperidin in Dimethylformamid bei 15 - 30 °C. Eine Abspaltung der DNP-Gruppe (2,4-Dinitrophenyl) gelingt z.B. auch mit einer etwa 3- bis 10%igen Lösung von 2-Mercaptoethanol in Dimethylformamid/Wasser bei 15-30 °C. Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole, wie Methanol oder Ethanol oder Amide wie Dimethylformamid. Die Hydrogenolyse wird in der Regel bei Temperaturen von etwa 0 bis 100 °C und einem Druck von etwa 1 bis 200 bar, bevorzugt bei 20 bis 30 °C und 1 bis 10 bar durchgeführt. Eine Hydrogenolyse der CBZ-Gruppe gelingt z.B. gut an 5- bis 10%igem Pd-Kohle in Methanol bei 20 -30 °C.

Als Aminoschutzgruppen, die mittels eines Reduktionssystems aus Metall und protonenabspaltendem Mittel abgespalten werden, sind beispielsweise (4-Nitro)-benzyl-oxycarbonyl, 2-Iod- oder 2,2,2-Trichlorethoxycarbonyl oder Phenacyloxycarbonyl, zu nennen.

Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Übergangsmetall, z.B. Zink, Zinn, Eisen oder Titan, während als Protonen-abspaltendes Mittel, z.B. Protonsäuren der vorstehend genannten Art, wie Salz- oder Essigsäure, $C_1$-$C_7$-Alkohole, wie Ethanol, und/oder Amine bzw. Ammoniak in Frage kommen. Solche Systeme sind beispielsweise Natrium/Ammoniak, Zink/Salz-oder Essigsäure oder Zink/Ethanol.

4-Nitrobenzyloxycarbonyl kann ferner z.B. mit einem Dithionit, wie Natriumdithionit, Phenacyloxycarbonyl und 2-Halogen-$C_2$-$C_7$-alkanoyl z.B. mit Hilfe eines nucleophilen Reagens, wie einem Thiolat, z.B. Natriumthiophenolat, oder Thioharnstoff und Base und sich anschließender Hydrolyse, und Allyl oder But-2-enyl, mit Hilfe eines Rhodium(III)halogenids, wie Rhodium(III) chlorid, gespalten werden.

Die Verbindungen der Formel (I), (II), (IIa) und (IIb) können als Enantiomeren- oder Diastereomerengemische anfallen.

Die Erfindung umfaßt sowohl die reinen Isomeren als auch die Gemische. Diese Gemische von Diastereomeren können nach gebräuchlichen Methoden, zum Beispiel durch selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung oder Trennung an optisch aktivem Säulenmaterial.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Verbindungen der Formel (I) und ihre Säureadditions-Salze weisen antimikrobielle, insbesondere starke antibakterielle und antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der

Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele:

Beispiel 1:

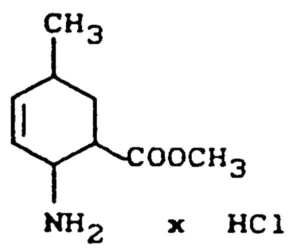

(I-1)

(Verfahren A)

Eine Lösung aus 10 g (0,05 Mol) 2-Carboxy-5-methyl-cyclohex-3-en-carbonsäuremethylester und 8 g (0,062 Mol) N,N-Diisopropyl-ethylamin in 30 ml Aceton wird bei -5 °C mit einer Lösung aus 5,4 g (0,05 Mol) Chlorameisensäureethylester in 15 ml Aceton über 30 Minuten versetzt.
Nach weiteren 30 Minuten bei 0 °C tropft man eine eisgekühlte Lösung aus 6,5 g (0,1 Mol) Natriumazid in 15 ml Wasser zu. Man läßt 15 Minuten bei 0 °C rühren und arbeitet dann mit Wasser/Toluol auf.
Die nach dem Trocknen und Einengen auf ein Restvolumen von ca. 50 ml erhaltene organische Phase wird zu 50 ml siedendem Toluol getropft und der Reaktionsverlauf IR-spektroskopisch verfolgt. Nach vollständiger Umlagerung in das Isocyanat engt man ein, nimmt den Rückstand in 50 ml Tetrahydrofuran und 50 ml 1 N Salzsäure auf und rührt 10 Stunden bei 40 °C.
Nach vollständigem Einengen unter vermindertem Druck erhält man 2,8 g (30 % der Theorie) 4-Methyl-6-carbomethoxy-2-cyclohexen-1-yl-aminhydrochlorid.
$^1$H-NMR Daten*)(DMSO, 200 MHz): $\delta$ = 1,00(3H), 1,50-1,70(1H), 1,85-2,00 und 2,15-2,35(2H), 2,85-3,00(1H), 3,68(3H), 3,80-3,85(2H), 5,70-5,90(2H)

Beispiel 2:

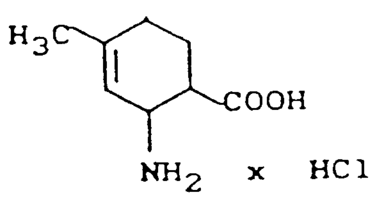

(I-2)

(Verfahren B)

2 g (7,8 mMol) tert-Butyl-(3-methyl-6-carboxy-2-cyclohexen-1-yl)-carbamat werden in 5 ml 1 n Salzsäure eingetragen. Nach 4 Stunden bei 50 °C engt man zur Trockne ein und erhält 1,3 g (87 % der Theorie) 3-Methyl-6-carboxy-2-cyclohexen-1-yl-aminhydrochlorid als weißen Feststoff mit dem Schmelzpunkt 156-

*) Die $^1$H-NMR-Spektren wurden in Dimethylsulfoxid (DMSO) mit Tetramethylsilan (TMS) als innerem Standard aufgenommen. Angegeben ist die chemische Verschiebung als $\delta$-Wert in ppm.

163 ° C.

In analoger Weise zu den in den Beispielen 1 und 2 beschriebenen Methoden und unter Berücksichtigung der Angaben in den Beschreibungen zu den erfindungsgemäßen Verfahren, werden die in der nachfolgenden Tabelle 1 aufgeführten Endprodukte der Formel (I)

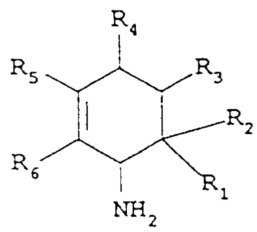

(I),

erhalten:

Tabelle 1

| Bsp-Nr. | $R^1$ | $R^2$ | $R^3$ | $R^4$ | $R^5/R^6$ | physikalische Konstanten |
|---|---|---|---|---|---|---|
| I-3 | H | COOH | H | $-CH_3$ | H, H | Fp: 190-193° C<br>x HCl / einheitliches cis-Diastereomeres |
| I-4 | H | COOH | H | H | " | Fp: 169-172° C<br>x HCl / einheitliches cis-Diastereomeres |
| I-5 | H | $COOCH_3$ | H | H | " | $^1$H-NMR*) ($CDCl_3$, 200 MHz):<br>δ= 1,65-2,20(6H), 2,57-2,68(1H), 3,60-3,70(2H), 3,71 (3H), 5,78(2H) |
| I-6 | H | COOH | $(CH_2-CH_2-CH_2-CH_2)-$ | | " | Fp: 185-205° C<br>x HCl |
| I-7 | H | $COOCH_3$ | H | H | " | $^1$H-NMR*) ($CDCl_3$, 200MHz):<br>δ= 1,90-2,4(4H), 3,03-3,17 (1H), 3,78(3H), 4,20(1H), 6,00(2H), 8,25-9,65(3H)<br>x HCl |
| I-8 | H | COOH | H | 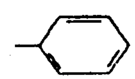 | " | Fp: 175-190° C<br>x HCl |
| I-9 | H | $COOCH_3$ | H | 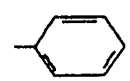 | " | $^1$H-NMR*) (DMSO, 200 MHz):<br>δ= 2,75-2,85(1H), 3,30-3,43 (1H), 3,65 und 3,68(3H), 5,75-6,00(2H), 7,10-7,40(5H) |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-10 | H | $COOCH_2CH_2OCH_3$ | H | $-CH_3$ | H, H | $^1$H-NMR*) (DMSO, 200 MHz): δ= 1,05(3H), 3,30(3H), 5,75-5,95(2H), 8,10-8,50(3H) x HCl |
| I-11 | H | COOH | H | $-C_3H_7$ | " | Fp: 180-185° C x HCl |
| I-12 | Cl | COOH | H | $-CH_3$ | " | Fp: 208-214° C x HCl |
| I-13 | H | COOH | H | $-C_2H_5$ | " | Fp: 168-178° C x HCl |
| I-14 | H | COOH | $C_2H_5$ | H | " | Fp: 140-150° C x HCl |
| I-15 | $CH_3$ | $COOCH_3$ | H | H | " | $^1$H-NMR*) (DMSO, 200 MHz): δ= 1,16 und 1,29(Isomere, 3H), 3,65(3H), 5,60-5,70 und 5,85-6,00(2H), 8,20-8,50 (3H) / x HCl |
| I-16 | H | COOH | $C_4H_9$ | H | " | Fp: 180-188° C x HCl |
| I-17 | H | CN | H | $-CH_3$ | " | $^1$H-NMR (DMSO, 200 MHz): δ= 1,05(3H), 1,80-2,10 u. 2,20-2,40(3H), 3,48(1H), 3,95(1H), 5,65-5,95(2H), 8,60-9,00(3H) / x HCl |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-18 | H | CN | H | $-C_2H_5$ | H, H | Fp: 130-135° C<br>x HCl |
| I-19 | Cl | CN | H | $-CH_3$ | " | 1H-NMR ($CDCl_3$, 200 MHz):<br>δ = 1,15(3H), 1,20-1,40 u.<br>1,85-2,55(5H), 3,60(1H),<br>5,60-5,85(2H) |
| I-20 | H | $NO_2$ | H | $-CH_3$ | 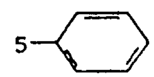 , 6-H | Fp: 215-221° C<br>x HCl |
| I-21 | H | $-CH=CH-C(=O)-OC_2H_5$ | H | $-CH_3$ | H, H | $^1$H-NMR*) ($CDCl_3$, 200 MHz):<br>δ= 1,02(3H), 1,29(3H),<br>4,18(2H), 5,55-6,00(3H),<br>6,83-7,10(1H) |
| I-22 | H | -CH=CH-COOH | H | H | " | Fp: 186-218° C<br>x HCl |
| I-23 | H | $-CH=CH-C(=O)-OC_2H_5$ | H | $-CH_3$ | " | Fp: 191-214° C<br>x HCl |
| I-24 | H | $-CH_2-OH$ | H | $-CH_3$ | " | Fp: 71-83° C<br>x HCl |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-25 | H | 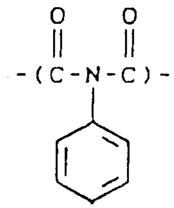 | | 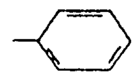 | H, H | Fp: 51-53° C<br>x $CF_3COOH$ |
| I-26 | H | 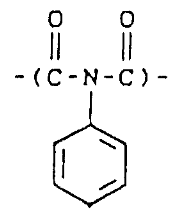 | | $-CH_3$ | " | Fp: 80-83° C<br>x $CF_3COOH$ |
| I-27 | H | 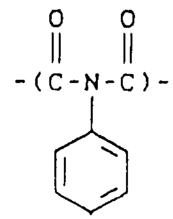 | | H | " | Fp: 75-81° C<br>x $CF_3COOH$ |
| I-28 | H | $-CH_2O-COCH_3$ | H | $-CH_3$ | " | $^1H$-NMR*) ($CDCl_3$, 200 MHz): δ = 1,08(3H), 2,10(3H), 4,02-4,28(2H), 5,76-6,00 (2H), 8,10-8,55(3H)<br>x HCl |
| I-29 | H | $-CO-N(CH_3)_2$ | H | H | " | IR: 3500-3350, 1655 $cm^{-1}$ |
| I-30 | H | $-CO-N(CH_3)_2$ | H | H | " | IR: 1670 $cm^{-1}$<br>x $CF_3COOH$ |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-31 | H | $-CO-N(CH_3)_2$ | H | $-CH_3$ | H, H | IR: 1670 $cm^{-1}$ x $CF_3COOH$ |
| I-32 | H | -CO-NH- 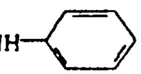 | H | $-CH_3$ | ″ | Fp: 111-127°C |
| I-33 | H | $-CO-OCH(CH_3)_2$ | H | $-CH_3$ | ″ | $^1$H-NMR*) ($CDCl_3$, 200 MHz): δ= 1,05(3H), 1,26(6H), 5,08(1H), 5,53-5,80(2H) |
| I-34 | H | $-CO-CH_2-$ 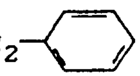 | H | $-CH_3$ | ″ | $^1$H-NMR*) ($CDCl_3$, 200 MHz): δ= 1,05(3H), 5,18(2H), 5,60-5,85(2H), 7,25-7,40(5H) |
| I-35 | F | COOH | H | $-CH_3$ | ″ | $^1$H-NMR*) ($CDCl_3$, 200 MHz): δ= 1,16(3H), 1,80-2,60(3H), 3,83-4,00(1H), 5,65-5,78 u. 6,00-6,13(2H) |
| I-36 | H | $-COO-C_4H_9$-n | H | $-CH_3$ | ″ | $^1$H-NMR*) (DMSO, 200 MHz): δ= 0,89(3H), 1,03(3H), 2,84-2,98(1H), 4,00-4,20(2H), 5,70-5,90(2H), 8,10-8,40(3H) |
| I-37 | H | $-COOC_4H_9$-n | H | $-CH_3$ | ″ | IR: 3500-3410, 1735 $cm^{-1}$ |
| I-38 | H | $-COOC_2H_5$ | H | $-CH_3$ | ″ | IR: 3500-3400, 1735 $cm^{-1}$ |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-39 | H | CN | H | $-C_2H_5$ | H, H | $^1H$-NMR*) ($CDCl_3$, 200 MHz): δ= 0,92(3H), 1,27-1,70(5H), 1,90-2,10(2H), 2,78-2,88 (1H), 3,53-3,60(1H), 5,65-5,80(2H) |
| I-40 | H | $-CH_2OH$ | H | $-CH_3$ | " | MS: m/z (rel.Int.): 141 (7), 99 (32), 83 (100) |
| I-41 | H | $-CH_2OH$ | H | H | " | $^1H$-NMR*) (DMSO, 200 MHz): δ= 1,30-2,10(5H), 5,63-5,82 u. 5,90-6,08(2H), 8,00-8,40 (3H) |
| I-42 | H | -COOH | H | H | 5-$CH(CH_3)_2$, 6-H | Fp: 189-205° C / x HCl |
| I-43 | H | -COOH | H | $-CH_3$ | H, H | Fp: 130-138° C Diastereomerenverhältnis 70:30/xHCl |
| I-44 | H | $-(C(=O)-O-CH_2-CH_2)-$ | | $-CH_3$ | " | $^1H$-NMR*) ($CDCl_3$, 200 MHz): δ= 0,80(3H), 1,80-2,20 u. 2,30-2,50 (4H), 2,90-3,05 (1H), 4,20-4,60(2H), 6,05-6,20 u. 6,30-6,40(2H), 7,30 (1H)/ x HCl |
| I-45 | H | $-COOC_2H_5$ | H | H | 5-$CH_3$, 6-H | $^1H$-NMR*) (DMSO, 200 MHz): δ= 0,93(3H), 1,21(3H), 2,55-2,65(1H), 4,10-4,25(2H), 5,70-6,63(2H), 8,10-8,30 (3H)/ x HCl |

<u>Tabelle 1</u> - Fortsetzung

| Bsp-Nr. | $R^1$ | $R^2$ | $R^3$ | $R^4$ | $R^5/R^6$ | physikalische Konstanten |
|---|---|---|---|---|---|---|
| I-46 | H | -COOH | H | $-CH_3$ | H, H | Drehwert: $[\alpha]_D^{20} = +76.6$ (C=0,3, $H_2O$)<br>x HCl<br>Enantiomere zu I-3 |
| I-47 | H | -COOH | H | $-CH_3$ | " | Drehwert: $[\alpha]_D^{20} = -84,1$ (C=0,3, $H_2O$)<br>x HCl<br>Enantiomere zu I-3 |
| I-48 | H | -CONH 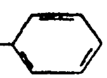 | H | $-CH_3$ | " | Fp: 128-133° C<br>x HCl |
| I-49 | H | $-COOCH(CH_3)_2$ | H | $-CH_3$ | " | $^1$H-NMR*) ($CDCl_3$, 200 MHz):<br>δ= 1,15(3H), 1,25(6H), 2,71-2,85(1H), 5,00-5,18(1H), 5,87-6,08(2H), 8,30-8,70 (3H) / x HCl |
| I-50 | H | 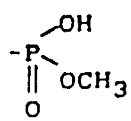 | H | $-CH_3$ | " | MS (FAB): 205 [$M^+$]<br>x HCl |
| I-51 | H | -COOH | H | $-CH_3$ | " | x $HO_2C-CH_2-CH-CO_2H$<br>OH<br>$^1$H-NMR (DMSO): δ = 1,00 (3H), 1,40 (m, 1H); 2,20-2,60 (6H); 3,75 (1H); 4,05 (1H); 5,60-5,90 (2H). |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-52 | H | -COOH | H | $-CH_3$ | H, H | • 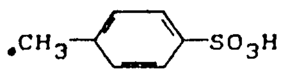 $^1$H-NMR (DMSO): δ = 1,00 (3H); 1,50 (m, 1H); 2,25 (3H); 2,80 (1H); 5,60-5,90 (2H); 7,10 (2H); 7,50 (2H). |
| I-53 | H | -COOH | H | $-CH_3$ | " | $\cdot CuSO_4$ Fp.: 210-219°C (Zers.) |
| I-54 | H | -COOH | H | $-CH_3$ | " | $\cdot HBr$ $^1$H-NMR (DMSO): δ = 1,00 (3H), 1,50 (1H); 2,80 (1H); 5,60-5,90 (2H); 7,90 (br, 3H). |
| I-55 | H | -COOH | H | $-CH_3$ | " | $\cdot CH_3CO_2H$ $^1$H-NMR (DMSO): δ = 0,95 (3H); 1,85 (3H); 5,60-5,80 (2H). |
| I-56 | H | -COOH | H | $-CH_3$ | " | $\cdot HCl$ Isomer zu I-3 $^1$H-NMR ($CD_3OD$): δ = 1,06 (3H); 1,83 (1H); 2,00 (1H); 2,38 (1H); 2,81 (1H); 4,08 (1H); 5,60 (1H); 6,00 (1H). |
| I-57 | H | $-CH_2-OH$ | H | $-CH_3$ | " | $\cdot HCl$ $^1$H-NMR (DMSO): δ = 1,00 (3H); 3,45 (2H); 5,70-5,90 (2H); 8,06 (3H). |
| I-58 | H | -COOH | H | $-CH_3$ | " | (Zwitterion) Fp.: 208-211°C |

<u>Tabelle 1</u> - Fortsetzung

| Bsp-Nr. | $R^1$ | $R^2$ | $R^3$ | $R^4$ | $R^5/R^6$ | physikalische Konstanten |
|---|---|---|---|---|---|---|
| I-59 | H | $-CO_2CH_3$ | H | $-CH_3$ | H, H | 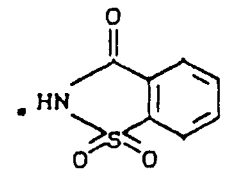 stark hygroskopisch |
| I-60 | H | $-CO_2CH_3$ | H | $-CH_3$ | '' | $\cdot HO_2CCH_2C(OH)-(CO_2H)CH_2CO_2H$ hygroskopisch |
| I-61 | H | $-CO_2CH_3$ | H | $-CH_3$ | '' | $\cdot HO_2CCO_2H$ Fp.: 152-158° C |
| I-62 | H | $-CO_2CH_3$ | H | $-CH_3$ | '' | 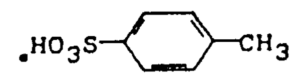 Fp.: 125-128° C |
| I-63 | H | $-CO_2CH_3$ | H | $-CH_3$ | '' | $\cdot CH_3CO_2H$ Fp.: 89,5-90° C |
| I-64 | H | $-CO_2(CH_2)_2OCH_3$ | H | $-CH_3$ | '' | $n_D^{20}$ 1,4763 |
| I-65 | H | $-CO_2CH_3$ | H | $-CH_3$ | '' | $n_D^{20}$ 1,4791 |
| I-66 | H | $-CO_2H$ | H | $-CH_3$ | '' | 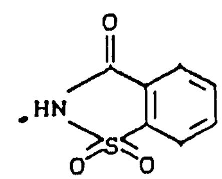 $^1$H-NMR (DMSO): $\delta$ = 1,00 (3H); 1,50 (1H); 2,00 (1H); 2,30 (1H); 2,85 (1H); 3,90 (1H); 5,60-5,95 (2H); 7,61 (5H). |

**Tabelle 1** - Fortsetzung

| Bsp-Nr. | $R^1$ | $R^2$ | $R^3$ | $R^4$ | $R^5/R^6$ | physikalische Konstanten |
|---|---|---|---|---|---|---|
| I-67 | H | $-CO_2H$ | H | $-CH_3$ | H, H | $\cdot HO_2CCO_2H$ $^1H$-NMR (DMSO): $\delta$ = 1,05 (3H), 1,45 (1H); 2,05 (1H), 2,31 (1H); 2,85 (1H); 3,80 (1H); 5,60-5,80 (2H). |
| I-68 | H | $-CO_2(CH_2)_3CH_3$ | H | $-CH_3$ | " | 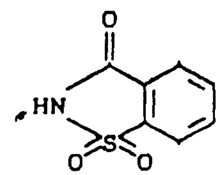 wachsartiger Feststoff |
| I-69 | H | $-CO_2CH_2CH(CH_3)_2$ | H | $-CH_3$ | " | $^1H$-NMR ($CDCl_3$): $\delta$ = 0,90-1,08 (9H); 2,60-2,73 (1H); 3,90 (2H); 5,60-5,80 (2H). |
| I-70 | H | $-CO_2CH_2CH(CH_3)_2$ | H | $-CH_3$ | " | $\cdot HCl$ $^1$-NMR (DMSO): $\delta$ = 0,85-1,05 (9H); 2,90-3,00 (1H); 3,90 (2H); 5,70-5,90 (2H); 5,20 (3H). |
| I-71 | H | $-CO_2H$ | H | $-CH_3$ | " | $\cdot Cu(OAc)_2$, MS (FAB): 155 [$M^+$ $-Cu(Ac)_2$] |
| I-72 | H | $-CO_2H$ | H | $-CH_3$ | " | $\cdot HO_2CCH_2CO_2H$ MS (FAB): 259 [$M^+$] |
| I-73 | H | $-CO_2H$ | H | $-CH_3$ | " | $\cdot HO_2C-CH=CH-CH=CH-CH_3$ MS (FAB): 267 [$M^+$] |
| I-74 | H | $-CO_2H$ | H | $-CH(CH_3)_2$ | " | $\cdot HCl$ Fp.: 203-206°C (Zers.) |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-75 | H | $-CO_2H$ | H | H | H, H | Fp.: 98° C (Zwitterion) |
| I-76 | H | $-CO_2H$ | $-CH_3$ | $-CH_3$ | ″ | .HCl Fp.: 133-135° C |
| I-77 | H | $-CO_2H$ | $-C_6H_5$ | $-CH_2CH_2(CH_3)_2$ | ″ | .HCl Fp.: 140-145° C |
| I-78 | H | $-CO_2^-Na^+$ | H | $-CH_3$ | ″ | Fp.: >250° C (Zersetzung) |
| I-79 | H | $-CO_2^-NH_4^+$ | H | $-CH_3$ | ″ | Fp.: >250° C (Zersetzung) |
| I-80 | H | $-CO_2CH_3$ | $-C_6H_5$ | $-CH_2CH(CH_3)_2$ | ″ | $^1$H-NMR ($CDCl_3$): δ = 0,75-0,95 (9H); 2,65-2,85 (1H); 3,75 (3H); 5,90 (1H); 7,15-7,40 (5H). |
| I-81 | H | $-CO_2H$ | H | H | ″ | $.HO_2CCO_2H$ Fp.: 147-150° C (Zersetzung) |
| I-82 | H | $-CO_2H$ | H | H | ″ | 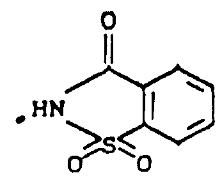 Fp.: 106-112° C (Zersetzung) |
| I-83 | H | $-CO_2H$ | $-C_6H_5$ | $-CH(CH_3)_2$ | ″ | .HCl Fp.: 245-248° C |
| I-84 | H | $-CO_2H$ | $-CH(CH_3)_2$ | $-CH_2CH(CH_3)_2$ | ″ | .HCl Fp.: 210-218° C |
| I-85 | H | -CN | $-CH(CH_3)_2$ | $-CH_2CH(CH_3)_2$ | ″ | .HCl Fp.: 164° C |
| I-86 | H | -CN | $-C_6H_5$ | $-CH(CH_3)_2$ | ″ | .HCL Fp.: 233-261° C |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-87 | H | $-CO_2C_2H_5$ | H | H | H,H | •HCl Fp.: 139-148° C |
| I-88 | H | $-CO_2CH(CH_3)_2$ | H | H | " | •HCl Fp.: 188-188,5° C |
| I-89 | H | $-CO_2C_6H_5$ | H | $-CH_3$ | " | Fp.: 117° C |
| I-90 | H | $-CO_2C_6H_5$ | H | $-CH_3$ | " | •HCl $^1$H-NMR (DMSO) $\delta$ = 1,00 (3H); 1,40 (1H); 2,80 (1H); 5,65-5,95 (2H); 7,20 (5H). |
| I-91 | H | $-CO_2CH_3$ | H | $-CH(CH_3)_2$ | " | •HCl MS: 197 [$M^+$ - HCl] |
| I-92 | H | $-CO_2$ 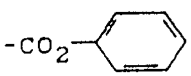 | H | $-CH_3$ | " | • 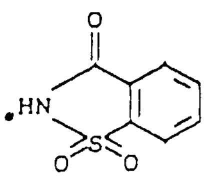 MS: 231 [$M^+$ - 183] |
| I-93 | H | $-CO_2H$ | H | H | " | •HCl (+)Enantiomer von I-4 Fp.: 210-213,5° C |
| I-94 | H | $-CO_2H$ | H | H | " | •HCl (-)Enantiomer von I-4 Fp.: 208-210° C |
| I-95 | H | $-CO_2H$ | H | 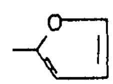 | " | •HCl MS: $M^+$ von 207 durch FAB-Spektroskopie |
| I-96 | H | $-CO_2CH_3$ | H | H | " | •HCl (Enantiomer) MS (FAB): 191 [$M^+$] |
| I-97 | H | $-CO_2CH_3$ | H | H | " | •HCl (Enantiomer) MS (FAB): 191 [$M^+$] |

<u>Tabelle 1</u> - Fortsetzung

| Bsp-Nr. | $R^1$ | $R^2$ | $R^3$ | $R^4$ | $R^5/R^6$ | | physikalische Konstanten |
|---|---|---|---|---|---|---|---|
| I-98 | H | $CO_2CH_2-C_6H_5$ | H | H | H, H | | $^1H$-NMR ($CDCl_3$): $\delta$ = 1,50 (2H); 2,60-2,70 (1H); 3,67 (1H); 5,15 (2H); 5,75 (2H); 7,32 (5H). |
| I-99 | H | $CO_2CH_2-2,4-Cl_2C_6H_3$ | H | $-CH_3$ | " | | $^1H$-NMR ($CDCl_3$): $\delta$ = 1,05 (d, 3H); 2,73-2,85 (1H); 5,22 (2H); 5,78 (2H); 7,22-7,40 (3H). |
| I-100 | H | $CO_2CH_2-C_6H_5$ | H | H | " | .HCl | MS: 231 [$M^+$ - 36] |
| I-101 | H | $CO_2CH_2-2,4-Cl_2C_6H_3$ | H | $-CH_3$ | " | .HCl | MS: 313 [$M^+$ - 36] |
| I-102 | H | $CO_2CH_2-2-ClC_6H_4$ | H | $-CH_3$ | " | | $^1H$-NMR ($CDCl_3$): $\delta$ = 1,05 (3H); 2,60 (2H); 2,65-2,77 (1H); 5,26 (2H); 5,56-5,80 (2H); 7,20-7,50 (4H). |
| I-103 | H | $CO_2CH_2-3-ClC_6H_4$ | H | $-CH_3$ | " | | $^1H$-NMR ($CDCl_3$): $\delta$ = 1,06 (3H); 2,68-2,80 (1H); 3,77 (1H); 5,15 (2H); 5,60-5,80 (2H); 7,3 (4H). |
| I-104 | H | $CO_2CH_2-4-ClC_6H_4$ | H | $-CH_3$ | " | | $^1H$-NMR ($CDCl_3$): $\delta$ = 1,05 (3H); 2,65-2,78 (1H); 5,15 (2H); 5,60-5,80 (2H); 7,32 (4H). |
| I-105 | H | $CO_2CH_2-4-NO_2C_6H_4$ | H | $-CH_3$ | " | | Fp.: 118-124°C |
| I-106 | H | $CO_2CH_2-2-ClC_6H_4$ | H | $-CH_3$ | " | .HCl | MS: 279 [$M^+$ - 36] |

**Tabelle 1 - Fortsetzung**

| Bsp-Nr. | $R^1$ | $R^2$ | $R^3$ | $R^4$ | $R^5/R^6$ | | physikalische Konstanten |
|---|---|---|---|---|---|---|---|
| I-107 | H | $CO_2CH_2$-3-$ClC_6H_4$ | H | $-CH_3$ | H,H | .HCl | MS: 279 [$M^+$ -36] |
| I-108 | H | $CO_2CH_2$-4-$ClC_6H_4$ | H | $-CH_3$ | " | .HCl | Fp.: 167° C |
| I-109 | H | $CO_2CH_2$-4-$NO_2C_6H_4$ | H | $-CH_3$ | " | .HCl | Fp.: 196-202° C |
| I-110 | H | $CO_2CH_2$-2-$ClC_6H_4$ | H | H | " | | $^1$H-NMR ($CDCl_3$): δ = 1,80-2,15 (4H); 2,65-2,85 (3H); 5,29 (2H); 5,78 (2H); 7,20-7,50 (4H). |
| I-111 | H | $CO_2CH_2$-3-$ClC_6H_4$ | H | H | " | | $^1$H-NMR ($CDCl_3$): δ = 1,75-2,15 (4H), 2,56 (2H); 2,65-2,75 (1H); 5,15 (2H); 5,79 (2H); 7,20-7,40 (4H). |
| I-112 | H | $CO_2CH_2$-4-$ClC_6H_4$ | H | H | " | | Fp.: 142° C |
| I-113 | H | $CO_2CH_2$-4-$NO_2C_6H_4$ | H | H | " | | $^1$H-NMR ($CDCl_3$): δ = 1,75-2,15 (6H); 5,28 (2H); 5,78 (2H); 7,55 (2H); 8,20 (2H). |
| I-114 | H | $CO_2CH_2$-2-$ClC_6H_4$ | H | H | " | .HCl | $^1$H-NMR (DMSO): δ = 1,95-2,12 (4H); 5,15-5,35 (2H); 5,65-6,05 (2H); 7,30-7,60 (4H); 8,25 (3H). |
| I-115 | H | $CO_2CH_2$-3-$ClC_6H_4$ | H | H | " | .HCl | $^1$H-NMR (DMSO): δ = 1,95-2,12 (4H); 3,10 (1H); 5,19 (2H); 7,39 (4H); 8,25 (3H). |
| I-116 | H | $CO_2CH_2$-4-$ClC_6H_4$ | H | H | " | .HCl | MS: 265 [$M^+$ - 36] |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-117 | H | $CO_2CH_2$-4-$NO_2C_6H_4$ | H | H | H,H | $^1$H-NMR (DMSO): δ = 1,90-2,15 (4H); 5,33 (2H); 5,70-6,05 (2H); 7,70 (2H); 8,20 (2H); 8,45 (3H). |
| I-118 | H | $CO_2CH_2$-3,5-$(OCH_3)_2C_6H_3$ | H | $-CH_3$ | " | $^1$H-NMR ($CDCl_3$): δ = 1,03 (3H); 3,79 (6H); 5,10 (2H); 5,58-5,80 (2H); 6,40-6,55 (3H). |
| I-119 | H | $CO_2CH_2$-2-$NO_2C_6H_4$ | H | $-CH_3$ | " | $^1$H-NMR ($CDCl_3$): δ = 1,05 (3H); 5,56 (2H); 5,60-5,85 (2H); 7,45-7,70 (3H); 8,10 (1H). |
| I-120 | H | $CO_2CH_2$-2,4-$Cl_2C_6H_3$ | H | H | " | $^1$H-NMR ($CDCl_3$): δ = 2,65-2,78 (1H); 2,70 (1H); 5,21 (2H); 5,78 (2H); 7,20-7,35 (3H). |
| I-121 | H | $CO_2CH_2$-3-$NO_2C_6H_4$ | H | H | " | MS: 276 [$M^+$] |
| I-122 | H | $CO_2CH_2$-3,5-$(OCH_3)_2C_6H_3$ | H | H | " | $^1$H-NMR ($CDCl_3$): δ = 3,79 (6H); 5,10 (2H); 5,80 (2H); 6,40-6,60 (3H). |
| I-123 | H | $CO_2CH_2$-3-$NO_2C_6H_4$ | H | $-CH_3$ | " | $^1$H-NMR ($CDCl_3$): δ = 1,05 (3H); 2,68-2,80 (1H); 5,28 (2H); 5,55-5,80 (2H); 7,50-7,75 (2H); 8,15-8,30 (2H). |

**Tabelle 1 - Fortsetzung**

| Bsp-Nr. | $R^1$ | $R^2$ | $R^3$ | $R^4$ | $R^5/R^6$ | physikalische Konstanten |
|---|---|---|---|---|---|---|
| I-124 | H | $CO_2CH_2-C_6H_5$ | H | $-CH(CH_3)_2$ | H,H | $^1H$-NMR ($CDCl_3$): δ = 0,90 (6H); 2,60-2,70 (1H); 5,17 (2H); 5,60-5,85 (2H); 7,35 (5H). |
| I-125 | H | $CO_2CH_2$-3,5-$(OCH_3)_2C_6H_3$ | H | $-CH_3$ | " | ,HCl $^1H$-NMR (DMSO): δ = 8,29 ($-NH_3^{\oplus}$, 3H). |
| I-126 | H | $CO_2CH_2$-2-$NO_2C_6H_4$ | H | $-CH_3$ | " | ,HCl $^1H$-NMR (DMSO): δ = 8,35 ($-NH_3^{\oplus}$, 3H). |
| I-127 | H | $CO_2CH_2$-2,4-$Cl_2C_6H_3$ | H | H | " | ,HCl $^1H$-NMR (DMSO): δ = 8,25 ($-NH_3^{\oplus}$, 3H). |
| I-128 | H | $CO_2CH_2$-3-$NO_2C_6H_4$ | H | H | " | ,HCl $^1H$-NMR (DMSO): δ = 8,40 ($-NH_3^{\oplus}$, 3H). |
| I-129 | H | $CO_2CH_2$-3,5-$(OCH_3)_2C_6H_3$ | H | H | " | ,HCl MS: 291 [$M^+$ - 36] |
| I-131 | H | $CO_2CH_2C_6H_5$ | H | $-CH(CH_3)_2$ | " | ,HCl $^1H$-NMR (DMSO): δ = 0,85 (6H); 1,55-2,10 (4H); 5,20 (2H); 5,80-6,00 (2H); 7,38 (5H); 8,35 (3H). |
| I-132 | H | $CO_2CH_2$-3,5-$Cl_2C_6H_3$ | H | H | " | $^1H$-NMR ($CDCl_3$): δ = 1,75-2,15 (6H); 2,65-2,80 (1H); 5,22 (2H); 5,78 (2H); 7,20-7,50 (3H). |
| I-133 | H | $CO_2CH_2$-2,6-$Cl_2C_6H_3$ | H | H | " | $^1H$-NMR ($CDCl_3$): δ = 1,75-2,15 (6H); 5,40 (2H); 5,75 (2H); 7,20-7,40 (3H). |

Tabelle 1 - Fortsetzung

| Bsp-Nr. | $R^1$ | $R^2$ | $R^3$ | $R^4$ | $R^5/R^6$ | physikalische Konstanten |
|---|---|---|---|---|---|---|
| I-134 | H | $CO_2CH_2$-3,5-$Cl_2C_6H_3$ | H | H | H,H | ·HCl $^1$H-NMR (DMSO): $\delta$ = 8,43 (-$NH_3^{\oplus}$, 3H). |
| I-135 | H | $CO_2CH_2$-2,6-$Cl_2C_6H_3$ | H | H | H,H | ·HCl $^1$H-NMR (DMSO): $\delta$ = 8,25 (-$NH_3^{\oplus}$, 3H). |

*) Die $^1$H-NMR-Spektren wurden in Deuterochloroform ($CDCl_3$) bzw. Dimethylsulfoxid (DMSO) mit Tetramethylsilan (TMS) als innerem Standard aufgenommen. Angegeben ist die chemische Verschiebung als $\delta$-Wert in ppm.

Anwendungsbeispiel:

Die erfindungsgemäßen Verbindungen wurden in den Applikationsarten iv - ip- sc und orale Gabe in den Modellen Mäuse-Candidose, Mäuse-Cryptococcose und Mäuse-Aspergillose auf in vivo-Wirksamkeit geprüft.

Beispielhaft sei der Ablauf der Mäuse-Candidose geschildert:

Männliche $CF_1$-SPF-Mäuse wurden mit 1-3x $10^6$ Sprosszellen von C.albicans pro Tier durch Injektion der Keimsuspension in phys. NaCl-Lösung (0,2 ml/Tier) in die Schwanzvene infiziert.

Nicht behandelte Kontrolltiere entwickeln unter diesen Infektionsbedingungen eine Nierencandidose und sterben zu 95-100 % der eingesetzten Tiere innerhalb 6 Tagen p.i. an dieser Infektion. Wurden infizierte Tiere täglich 2 mal, beginnend mit dem Tag der Infektion, mit den erfindungsgemäßen Verbindungen oral oder parenteral in Dosen 2 x 5 bis 2 x 50 mg/kg KG über 2-5 Tage behandelt, so überleben >80 - 100 %

der Tiere die Infektion in gutem Zustand.
Die C.albicans-Keimzahlen in den Nieren der infizierten und behandelten Tiere liegen am 4. Tag p.i durchschnittlich um 2-3 Zehnerpotenzen unter den von unbehandelten, infizierten Kontrolltieren.

Vergleichbare Wirkungen können auch in den Versuchsmodellen Mäuse-Cryptococcose und Mäuse-Aspergillose erzielt werden.
Nach orientierenden Untersuchungen zur Pharmakokinetik der erfindungsgemäßen Verbindungen an Mäuse nach oraler Gabe von 25 mg/kg KG werden diese intestinal rasch und nahezu vollständig resorbiert. Es resultieren maximale Serumkonzentrationen von 20- >30 mcg/ml.

Die Wirkstoffe werden renal innerhalb von 12 Std. post. appl. eliminiert. Die Urinkonzentrationen erreichen Werte zwischen 10 und > 30 mcg/ml.

In der folgenden Tabelle sind die in vivo-Wirkungen einiger Verbindungen beispielhaft am Modell der Mäusecandidose dargestellt:

| Beispiel-Nr. | Dosis oral mg/kg | Zahl der Überlebenden Tiere am 6. Tag p.i. |
|---|---|---|
| Kontrolle | 0 | 1/10 |
| I 24 | 2 x 25 | 8/10 |
| I100 | 2 x 25 | 10/10 |
| I 87 | 2 x 10 | 10/10 |
| I 45 | 2 x 50 | 7/10 |

**Patentansprüche**

1. Substituierte 2-Cyclohexen-1-yl-amin-Derivate der Formel (I)

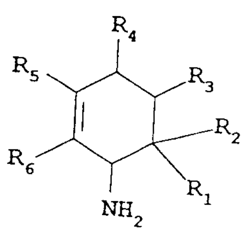

(I),

in welcher

R¹ für Wasserstoff, geradkettiges oder verzweigtes Alkyl mit 1 bis 6 Kohlenstoffatomen oder Fluor, Chlor oder Brom steht,

$R^2$ für Formyl, geradkettiges oder verzweigtes Hydroxyalkyl mit 1 bis 8 Kohlenstoffatomen im Alkylteil, Cyano, Nitro oder für einen der Reste

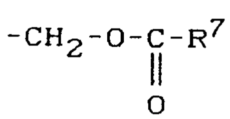 , 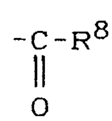

oder $-CH=CH-R^9$ steht,

$R^3$ , $R^4$ , $R^5$ und $R^6$ gleich oder verschieden sind und jeweils für Wasserstoff, jeweils geradkettiges oder verzweigtes Alkyl oder Alkoxy mit 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, Alkoxyalkyloxy mit jeweils 1 bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, $C_1$-$C_4$-

Alkyl, $C_1$-$C_4$-Alkoxy, $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino,
weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Halogen, Nitro, Cyano, Amino, $C_1$-$C_4$-Alkyl, $C_1$-$C_4$-Alkoxy oder $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino, weiterhin für Fluor, Chlor oder Brom stehen,
wobei mindestens zwei der Reste $R^3$ , $R^4$ , $R^5$ oder $R^6$ für Wasserstoff stehen,

$R^7$ für jeweils geradkettiges oder verzweigtes Alkyl oder Alkoxy mit 1 bis 6 Kohlenstoffatomen steht,

$R^8$ für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, unsubstituiertes oder einfach bis mehrfach, gleich oder verschieden durch Halogen substituiertes Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine Gruppe -O-Z-$NR^{11}$ $R^{12}$ , -$NHR^{10}$ , -$NR^{11}$ $R^{12}$ oder -OM steht,

$R^9$ für Formyl, Cyano oder für die Gruppe

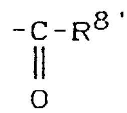

steht,

$R^{10}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{11}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{12}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten infrage kommen,

M für Wasserstoff oder für ein Äquivalent eines entsprechenden Alkalimetall-, Erdalkalimetall- oder Ammoniumkations steht und

Z für eine geradkettige oder verzweigte Alkylkette mit 1 bis 8 Kohlenstoffatomen steht

oder

$R^2$ und $R^3$ gemeinsam für einen der Reste

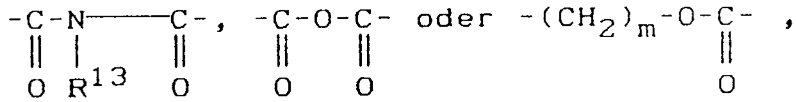

verbrückt über die Positionen 6 und 5, stehen,

worin

$R^{13}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen und

m für eine Zahl 1 oder 2 steht,

oder

$R^4$ und $R^5$ gemeinsam für eine Alkylkette mit 3- oder 4-Kohlenstoffatomen steht, die über die Positionen 4 und 3 verbunden ist sowie deren Säureadditionssalze und Metallsalz-Komplexe zur Anwendung in einem Verfahren zur therapeutischen Behandlung des menschlichen oder tierischen Körpers.

**2.** Substituierte 2-Cyclohexen-1-yl-amin-Derivate gemäß Anspruch 1 zur Anwendung in einem Verfahren zur therapeutischen Behandlung des menschlichen oder tierischen Körpers in Form ihrer enantiomerenreinen Verbindungen.

**3.** Verwendung von substituierten 2-Cyclohexen-1-yl-amin-Derivaten gemäß den Ansprüchen 1 und 2 bei der Herstellung von Arzneimitteln zur Bekämpfung von Krankheiten.

## Claims

**1.** Substituted 2-cyclohexen-1-yl-amine derivatives of the formula (I)

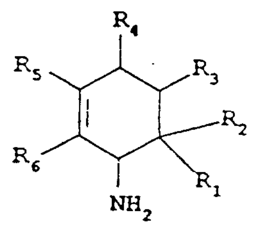

(I)

in which

$R^1$ represents hydrogen, straight-chain or branched alkyl having 1 to 6 carbon atoms or fluorine, chlorine or bromine,

$R^2$ represents formyl, straight-chain or branched hydroxyalkyl having 1 to 8 carbon atoms in the alkyl moiety, cyano, nitro or one of the radicals

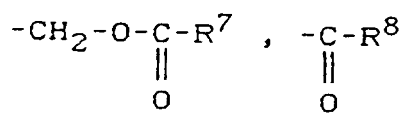

or $-CH=CH-R^9$ ,

$R^3$, $R^4$, $R^5$ and $R^6$ are identical or different and in each case represent hydrogen, alkyl or alkoxy having 1 to 8 carbon atoms, which is in each case straight-chain or branched, alkenyl, alkinyl, alkenyloxy or alkinyloxy in each case having 2 to 8 carbon

atoms, which is in each case straight-chain or branched, alkoxyalkyloxy in each case having 1 to 8 carbon atoms in the individual alkyl moieties, aryl or aralkyl in each case having 6 to 10 carbon atoms in the aryl moiety and optionally 1 to 4 carbon atoms in the alkyl moiety and in each case unsubstituted or monosubstituted to pentasubstituted in the aryl moiety by identical or different substituents, suitable aryl substituents being: halogen, nitro, cyano, amino, $C_1$-$C_4$-alkyl, $C_1$-$C_4$-alkoxy or $C_1$-$C_4$-alkylthio, halogeno-($C_1$-$C_4$)-alkyl, halogeno-($C_1$-$C_4$)-alkoxy or halogeno-($C_1$-$C_4$)-alkylthio in each case having 1 to 9 identical or different halogen atoms, and di-($C_1$-$C_4$)-alkylamino,

furthermore represent a heterocyclic 5- or 6-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl which is unsubstituted or monosubstituted to trisubstituted by identical or different substituents and optionally bonded via a methylene group, suitable substituents for the heterocycle in each case being: halogen, nitro, cyano, amino, $C_1$-$C_4$-alkyl, $C_1$-$C_4$-alkoxy or $C_1$-$C_4$-alkylthio, halogeno-($C_1$-$C_4$)-alkyl, halogeno-($C_1$-$C_4$)-alkoxy or halogeno-($C_1$-$C_4$)-alkylthio in each case having 1 to 9 identical or different halogen atoms, and di-($C_1$-$C_4$)-alkylamino,

furthermore represent fluorine, chlorine or bromine,

where at least two of the radicals $R^3$, $R^4$, $R^5$ or $R^6$ represent hydrogen,

$R^7$ represents alkyl or alkoxy having 1 to 6 carbon atoms, which is in each case straight-chain or branched,

$R^8$ represents hydroxyl, straight-chain or branched hydroxyalkyloxy having 1 to 8 carbon atoms, straight-chain or branched halogenoalkyloxy having 1 to 8 carbon atoms and 1 to 17 identical or different halogen atoms, cycloalkyloxy having 3 to 6 carbon atoms and unsubstituted or monosubstituted to polysubstituted by identical or different halogen substituents, alkoxy or alkylthio having 1 to 6 carbon atoms, which is in each case straight-chain or branched, straight-chain or branched alkoxyalkyloxy in each case having 1 to 6 carbon atoms in the alkoxy or alkyl moiety, aryloxy, arylthio, aralkyl or aralkyloxy in each case having 6 to 10 carbon atoms in the aryl moiety and optionally 1 to 8 carbon atoms in the alkyl moiety and in each case unsubstituted or monosubstituted to pentasubstituted in the aryl moiety by identical or different substituents, suitable aryl substituents being the abovementioned aryl substituents, or represents a group -O-Z-$NR^{11}R^{12}$, -$NHR^{10}$, -$NR^{11}R^{12}$ or -OM,

$R^9$ represents formyl, cyano or the group

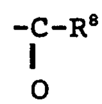

$R^{10}$ represents hydrogen, straight-chain or branched alkyl having 1 to 6 carbon atoms or aryl having 6 to 10 carbon atoms, which is unsubstituted or monosubstituted to pentasubstituted by identical or different substituents, suitable aryl substituents being the abovementioned aryl substituents,

$R^{11}$ represents straight-chain or branched alkyl having 1 to 6 carbon atoms or aryl having 6 to 10 carbon atoms, which is unsubstituted or monosubstituted to pentasubstituted by identical or different substituents, suitable aryl substituents being the abovementioned aryl substituents,

$R^{12}$ represents straight-chain or branched alkyl having 1 to 6 carbon atoms or aryl having 6 to 10 carbon atoms, which is unsubstituted or monosubstituted to pentasubstituted by identical or different substituents, suitable aryl substituents being the abovementioned aryl substituents,

M represents hydrogen or an equivalent of an appropriate alkali metal, alkaline earth metal or ammonium cation and

Z represents a straight-chain or branched alkyl chain having 1 to 8 carbon

atoms,

or

$R^2$ and $R^3$ together represent one of the radicals

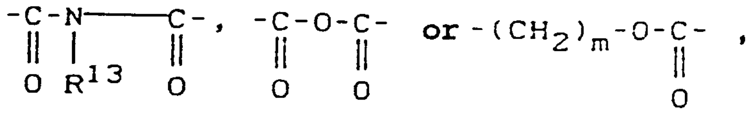

or ,

bridged via the positions 6 and 5,

in which

$R^{13}$ represents hydrogen, straight-chain or branched alkyl having 1 to 6 carbon atoms or aryl having 6 to 10 carbon atoms, which is unsubstituted or monosubstituted to pentasubstituted by identical or different substituents, suitable aryl substituents being the abovementioned aryl substituents and

m represents a number 1 or 2,

or

$R^4$ and $R^5$ together represent an alkyl chain having 3 or 4 carbon atoms, which is bonded via the positions 4 and 3

and their acid addition salts and metal salt complexes for use in a method for the therapeutic treatment of the human or animal body.

2. Substituted 2-cyclohexen-1-yl-amine derivatives according to Claim 1 for use in a method for the therapeutic treatment of the human or animal body, in the form of their enantiomerically pure compounds.

3. Use of substituted 2-cyclohexen-1-yl-amine derivatives according to Claims 1 and 2 in the production of medicaments for the control of diseases.

**Revendications**

1. Dérivés substitués de 2-cyclohexène-1-yl-amine de formule (I)

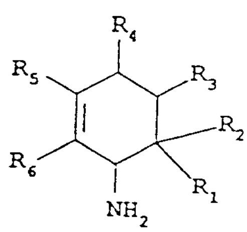

(I),

dans laquelle

$R^1$ représente de l'hydrogène, un groupe alkyle linéaire ou ramifié ayant 1 à 6 atomes de carbone ou du fluor, du chlore ou du brome,

$R^2$ est un groupe formyle, un groupe hydroxyalkyle linéaire ou ramifié ayant 1 à 8 atomes de carbone dans la partie alkyle, un groupe cyano, nitro ou l'un des restes

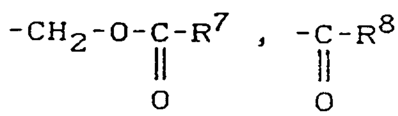

,

ou $-CH=CH-R^9$

$R^3$, $R^4$, $R^5$ et $R^6$ sont égaux ou différents et représentent chacun de l'hydrogène, un groupe alkyle ou alkoxy linéaire ou ramifié ayant 1 à 8 atomes de carbone, un groupe alcényle, alcynyle, alcényloxy ou alcynyloxy linéaire ou ramifié ayant chacun 2 à 8 atomes de carbone, un groupe alkoxyalkyloxy ayant 1 à 8 atomes de carbone dans les parties alkyle individuelles, un groupe aryle ou aralkyle ayant chacun 6 à 10 atomes de carbone dans la partie aryle et le cas échéant 1 à 4 atomes de carbone dans la partie alkyle, chacun étant non substitué dans la partie aryle ou porteur de 1 à 5 substituants égaux ou différents, en considérant comme substituants de la partie aryle : un halogène, un radical nitro, cyano, amino, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkylthio en $C_1$ à $C_4$ ayant chacun 1 à 9 atomes d'halogènes identiques ou différents et di-(alkyle en $C_1$ à $C_4$)amino,

en outre un groupement hétérocyclique pentagonal ou hexagonal non substitué ou portant 1 à 3 substituants identiques ou différents et lié le cas échéant par l'intermédiaire d'un groupe méthylène, de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxadiazolyle, thiazolyle, isothiazolyle, 1,2,3-, 1,2,4-, 1,2,5- ou 1,3,4-thiadiazolyle, pyridyle, pyridazinyle, pyrimidinyle et pyrazinyle, en considérant comme substituants de chaque hétérocycle : un halogène, un radical nitro, cyano, amino, alkyle en $C_1$ à $C_4$, alkoxy en $C_1$ à $C_4$ ou alkylthio en $C_1$ à $C_4$, halogénalkyle en $C_1$ à $C_4$, halogénalkoxy en $C_1$ à $C_4$, halogénalkylthio en $C_1$ à $C_4$ ayant chacun 1 à 9 atomes d'halogènes identiques ou différents et di-(alkyle en $C_1$ à $C_4$)amino, en outre du fluor, du chlore ou du brome,

deux au moins des restes $R^3$, $R^4$, $R^5$ ou $R^6$ représentant de l'hydrogène,

$R^7$ est un groupe alkyle ou alkoxy linéaire ou ramifié ayant 1 à 6 atomes de carbone,

$R^8$ est un groupe hydroxy, un groupe hydroxyalkyloxy linéaire ou ramifié ayant 1 à 8 atomes de carbone, un groupe halogénalkyloxy linéaire ou ramifié ayant 1 à 8 atomes de carbone et 1 à 17 atomes d'halogènes identiques ou différents, un groupe cycloalkyloxy de 3 à 6 atomes de carbone non substitué ou portant un ou plusieurs substituants halogéno identiques ou différents, un groupe alkoxy ou alkylthio linéaire ou ramifié ayant chacun 1 à 6 atomes de carbone, un groupe alkoxyalkyloxy linéaire ou ramifié ayant chacun 1 à 6 atomes de carbone dans la partie alkoxy ou alkyle, un groupe aryloxy, arylthio, aralkyle ou aralkoxy ayant chacun 6 à 10 atomes de carbone dans la partie aryle et le cas échéant 1 à 8 atomes de carbone dans la partie alkyle, non substitué dans la partie aryle ou portant chacun 1 à 5 substituants identiques ou différents, en considérant comme substituants de la partie aryle les substituants indiqués ci-dessus pour la partie aryle, ou un groupe $-O-Z-NR^{11}R^{12}$, $-NHR^{10}$, $-NR^{11}R^{12}$ ou -OM,

$R^9$ est un groupe formyle, cyano ou le groupe

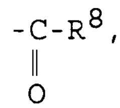

$R^{10}$ est de l'hydrogène, un groupe alkyle linéaire ou ramifié ayant 1 à 6 atomes de carbone ou un groupe aryle de 6 à 10 atomes de carbone non substitué ou portant 1 à 5 substituants identiques ou différents, en considérant comme substituant de la partie aryle les substituants indiqués ci-dessus pour le groupe aryle,

$R^{11}$ est un groupe alkyle linéaire ou ramifié ayant 1 à 6 atomes de carbone ou un groupe aryle de 6 à 10 atomes de carbone non substitué ou portant 1 à 5 substituants identiques ou différents, en considérant comme substituants de la partie aryle les substituants du groupe aryle indiqués ci-dessus,

$R^{12}$ est un groupe alkyle linéaire ou ramifié ayant 1 à 6 atomes de carbone ou un groupe aryle de 6 à 10 atomes de carbone non substitué ou portant 1 à 5

substituants identiques ou différents, en considérant comme substituants de la partie aryle les substituants indiqués ci-dessus pour le groupe aryle,

M est de l'hydrogène ou un équivalent d'un cation de métal alcalin, de métal alcalino-terreux ou d'ammonium correspondant et

Z est une chaîne alkylique linéaire ou ramifiée ayant 1 à 8 atomes de carbone,

ou bien

$R^2$ et $R^3$ forment conjointement l'un des restes

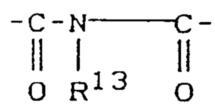 , 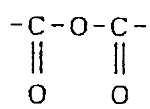 ou 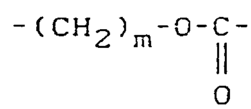 ,

ponté par l'intermédiaire des positions 6 et 5,

où

$R^{13}$ est de l'hydrogène, un groupe alkyle linéaire ou ramifié ayant 1 à 6 atomes de carbone ou un groupe aryle de 6 à 10 atomes de carbone non substitué ou portant 1 à 5 substituants identiques ou différents, en considérant comme substituants du groupe aryle les substituants indiqués ci-dessus pour le groupe aryle et

m est le nombre 1 ou 2,

ou bien

$R^4$ et $R^5$ forment conjointement une chaîne alkylique de 3 ou 4 atomes de carbone qui est liée par l'intermédiaire des positions 4 et 3

ainsi que leurs sels d'addition d'acides et leurs complexes de sels métalliques, destinés à être utilisés dans un procédé de traitement thérapeutique du corps humain ou animal.

**2.** Dérivés substitués de 2-cyclohexène-1-ylamine suivant la revendication 1, destinés à être utilisés dans un procédé de traitement thérapeutique du corps humain ou animal, sous forme de leurs composés de pureté énantiomérique.

**3.** Utilisation de dérivés substitués de 2-cyclohexène-1-ylamine suivant les revendications 1 et 2 dans la préparation de médicaments destinés à combattre des maladies.